# EUROPEAN PATENT SPECIFICATION

(11) **EP 3 450 622 B1**
(45) Date of publication and mention of the grant of the patent: **26.06.2024**
(21) Application number: 18190062.2
(22) Date of filing: 21.08.2018
(51) Int. Cl.: D21C 1/02, C08H 8/00, C13K 1/02, C12P 7/08, C12P 7/10

(54) **METHOD AND APPARATUS FOR THE TREATMENT OF LIGNOCELLULOSIC BIOMASSES**
VERFAHREN UND VORRICHTUNG ZUR BEHANDLUNG VON LIGNOCELLULOSEBIOMASSEN
PROCÉDÉ ET APPAREIL DE TRAITEMENT DE BIOMASSES LIGNOCELLULOSIQUES

(30) Priority: 01.09.2017 IT 201700098245
(43) Date of publication of application: 06.03.2019
(73) Proprietor: Water And Soil Remediation S.r.l., 46010 Curtatone, Frazione Levata MN (IT)
(72) Inventor: PRANDI, Alberto, 46100 MANTOVA (IT)
(74) Representative: Modiano, Micaela Nadia

(56) References cited:
- JP-A- 2010 162 498
- US-A1- 2009 098 617
- US-A1- 2013 134 351
- US-A1- 2016 244 788
- US-A1- 2017 210 826

## Description

The present invention relates to a method and an apparatus for treating lignocellulosic biomasses, particularly for separating cellulose from lignin, preparatory for subsequent transformation of cellulose into simple sugars.

Currently 99% of fermentation-derived ethanol originates from starches, mainly grains of corn and cereals (North America and Europe) or sugarcane (Brazil).

Enzyme hydrolysis of polysaccharides contained in starches is easy, since they can be attacked directly by enzymes because they are pure and lack casing-like structures in which they are enclosed.

Polysaccharides of celluloses (plants, hays, grasses) are instead enclosed by a lignin casing, which is impermeable to enzymes. For this reason, on lignocellulosic biomasses it is common to perform a pretreatment aimed at destroying the lignin casing and thus make the celluloses available to the hydrolysis enzymes.

The pretreatment method currently most used to break the lignin casing of lignocellulosic biomasses, separating celluloses from lignin, resorts to the so-called "steam explosion", with or without the addition of acids or other chemical compounds, and consists of the rupture of the structure of the lignin produced in the biomass by means of a sudden expansion of steam injected for this purpose with the biomass.

Steam explosion was introduced for the first time by Mason et al. in 1926. In the current method for the pretreatment of lignocellulosic biomasses that uses steam explosion, the biomass is treated with steam heated to temperatures usually comprised between 180°C and 240°C, usually around 200°C, at a pressure usually comprised between 10 and 35 bar (1 and 3.5 MPa), usually around 15 bar (1.5 MPa). Incubation at the temperature for a variable time is followed by explosive decompression of the mixture of biomass and steam by transferring it into a vessel at a pressure that is close to atmospheric pressure, which leads to rupture of the rigid structure of the fibers of the biomass. The sudden pressure release defibrillates the cellulose bundles, leading to greater accessibility of the cellulose for subsequent enzyme hydrolysis and fermentation. Depending on the incubation time and temperature, steam explosion can lead to the forming of small cracks in the structure of the wood up to total defibrillation of the wood fibers. It is also known that this method can be accelerated by means of the addition of diluted acids, such as sulfuric acid or nitric acid, which facilitate the treatment of the biomass.

More precisely, in the manner in which it is practiced commonly, steam explosion uses an apparatus that pumps the biomass into a screw feeder, in the intermediate part of which saturated steam at a temperature comprised between 180°C and 240°C, usually approximately 200°C, is injected. At the end of the screw feeder, vaporization (flash) occurs by means of the cyclic opening of a valve and the introduction of the superheated mixture of biomass and steam into an environment at reduced pressure.

However, this process has some drawbacks. The production of steam required by the process entails high energy consumption and thermal levels that cannot be met by ordinary heat sources, such as for example remote heating or hot fluids that originate from other processes.

Moreover, the use of very high temperatures can lead to a breakdown of the molecules of the biomass with the release of byproducts that inhibit the subsequent fermentation stages. Various strategies are known for limiting the formation and the effects of byproducts that inhibit enzyme processes. For example, it is possible to limit the process to biomasses that are easier to treat and generate fewer inhibitors, or perform conditioning/detoxication processes by means of the use of chemical additives, for example alkali. These methods, however, introduce further limitations, such as the use of chemical substances and the addition of further steps to the process,

Documents JP2010162498, US2017210826 and US2009098617 relate to methods and apparatuses for pre-treating a lignocellulosic material with hot water.

The aim of the present invention is to eliminate the drawbacks described above in the steam explosion process by providing a new method for the treatment of a lignocellulosic biomass that is more convenient from an energy standpoint and does not require the use of chemical substances to facilitate the breakdown of the structure of lignin.

Within this aim, an object of the invention is to provide a method that can be performed at lower temperatures than the steam explosion process commonly performed in the background art.

Another object of the invention is to provide a process the thermal requirements of which can be met by ordinary heat sources, such as remote heating or hot fluids that originate from other processes.

A still further object of the present invention is to allow effective pretreatment of the lignocellulosic biomass, limiting the degree of breakdown of the molecules of the biomass and the forming of byproducts that inhibit the subsequent fermentation stages.

Another object of the present invention is to provide an apparatus for performing the method according to the invention that is highly reliable, relatively easy to provide and at competitive costs.

This aim, as well as these and other objects which will become better apparent hereinafter, are achieved by a method for the treatment of a lignocellulosic biomass comprising the steps of:
a) providing a mixture comprising water and the lignocellulosic biomass having a water:biomass weight ratio lower than or equal to 2:1, preferably lower than 1:1;
b) heating and maintaining said mixture comprising water and the lignocellulosic biomass at a temperature comprised between 80°C and 135°C;
c) vaporizing at an absolute pressure comprised between 5 and 250 millibar (0.5 and 25 kPa) said mixture comprising water and the lignocellulosic biomass.

The aim and objects of the present invention are also achieved by an apparatus for treating a lignocellulosic biomass according to the method described above, comprising:
a) movement means adapted to move said mixture comprising water and the lignocellulosic biomass in a conveyance duct by applying a partial vacuum;
b) a cooking vessel connected to said conveyance duct and comprising heating means adapted to heat and maintain the temperature of said mixture comprising water and the lignocellulosic biomass;
c) a vessel at a pressure that is lower than atmospheric pressure, which is connected to said conveyance duct downstream of said cooking vessel, adapted to vaporize said mixture comprising water and the lignocellulosic biomass.

Further characteristics and advantages of the invention will become better apparent from the description of a preferred but not exclusive embodiment of the method and of the apparatus according to the invention, illustrated by way of nonlimiting example in the accompanying drawings, wherein:
Figure 1 is a block diagram of the method according to the invention according to a particular embodiment;
Figure 2 is a view of an apparatus that provides the method according to the invention according to a particular embodiment.

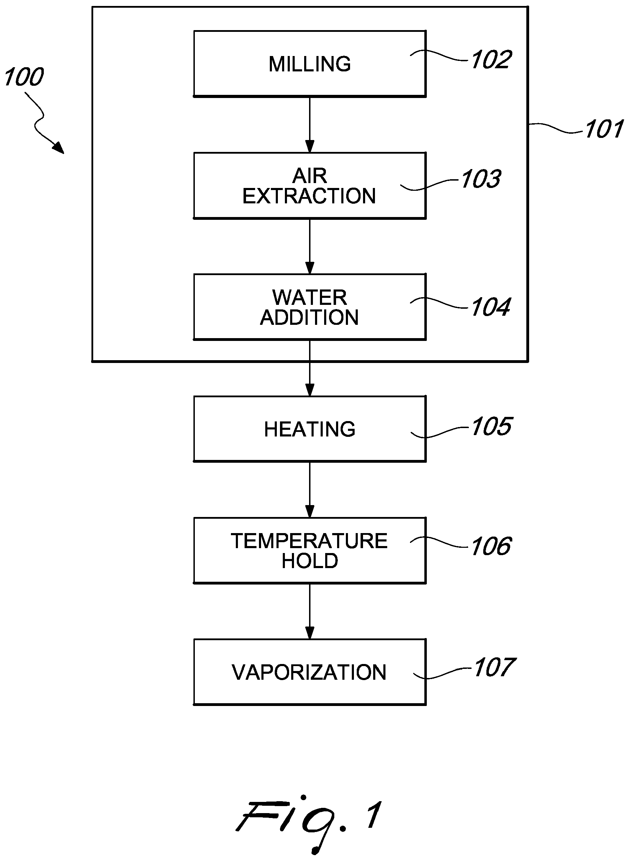

With reference to Figure 1, the method 100 for treating a lignocellulosic biomass comprises the steps of:
a) providing 101 a mixture comprising water and the lignocellulosic biomass having a water:biomass weight ratio lower than or equal to 2:1, preferably lower than 1:1;
b) heating 105 and maintaining 106 said mixture comprising water and the lignocellulosic biomass at a temperature comprised between 80°C and 135°C;
c) vaporizing 107 at an absolute pressure comprised between 5 and 250 millibar (0.5 and 25 kPa) said heated mixture comprising water and the lignocellulosic biomass.

It should be specified that the water:biomass ratio is considered as weight of water:weight of biomass expressed as dry substance.

With respect to the steam explosion process, where it is the steam that expands (and the water, only partially) from the initial pressure to atmospheric pressure, the present invention relates to an expansion of greater proportions, induced by the state transition from liquid to steam of the water contained and introduced in the biomass.

More precisely, instead of injecting steam into the biomass, superheated water in the liquid state is added, in a quantity that is preset in order to obtain a given concentration on the dry substance, preferably in such a manner as to facilitate its penetration in the lignin casing as will be described in detail hereinafter; this mixture is kept compressed and simultaneously heated, where necessary, until temperatures between comprised between 80°C and 135°C, are achieved, before proceeding with vaporization (flash). This vaporization occurs in a chamber at a pressure that is lower than atmospheric pressure, wherein the water contained in the mixture vaporizes, generating an important increase in volume.

In a preferred embodiment, the step 101 of providing the mixture comprising water and the lignocellulosic biomass comprises the steps of:
a1) milling 102 the lignocellulosic biomass;
a2) extracting 103 air from the milled lignocellulosic biomass;
a3) adding 104 optionally heated water to the lignocellulosic biomass from which the air has been extracted until a water:biomass ratio of less than or equal to 2:1, preferably less than 1:1, is obtained.

In a preferred embodiment of the method according to the invention, the lignocellulosic biomass is milled into fragments with a size of less than 0.5 mm. Advantageously, the milling step 102 facilitates the exposure of the biomass to the subsequent steps provided by the present method and is performed for example by using a mill, preferably capable of shredding finely the biomass to the dimensions mentioned above.

The lignocellulosic biomass is saturated with air in its interstitial volumes, including the volumes enclosed by lignin casings. This air can hinder or even prevent the entry of water into the interstitial volumes. Therefore, in a preferred embodiment of the method according to the present invention, before adding 104 the necessary amount of water to the possibly milled biomass, the air is extracted 103 from the lignocellulosic biomass, preferably by applying vacuum. This treatment for extracting the air from the biomass facilitates a more effective migration of water inside the lignin alveoles and leads to a higher efficiency of the method according to the invention.

As mentioned earlier, in the present method steam is not used as the driving force of the steam explosion, but liquid water is used instead, added to the lignocellulosic biomass, preferably after the application of vacuum. This difference entails an important advantage with respect to the standard steam explosion method, since it is possible to save both the energy required for water vaporization and the costs associated with a steam boiler.

The water is added 104 to the lignocellulosic biomass in order to obtain a mixture that has a ratio of 2:1 or less in terms of water:dry biomass. Although in the method according to the invention it is possible to use ratios up to 2:1, preferably a water:biomass of less than 1:1 is used.

In another preferred embodiment of the method according to the invention, the water of the mixture comprising water and the lignocellulosic biomass is desalinated water. Desalination of the water has the purpose of lowering its osmotic pressure and thus facilitate its penetration in the lignin casings; it is in fact the water that has penetrated into the lignin casings which, when subjected to reduced pressure, vaporizes (flash), expands and breaks up the lignin structures.

It should be specified that the term "desalinated" or "desalinized" is understood here to reference water commonly known as deionized, i.e., water of a quality that is suitable to supply a boiler for the production of steam; in terms of composition, it has a saline content of less than 20 milligrams/liter.

In another preferred embodiment of the method according to the invention, the water of the mixture comprising water and the lignocellulosic biomass receives the addition of carbon dioxide. The addition of carbon dioxide has the purpose of further increasing the expansion rate during the transition from liquid to steam; this leads to a mechanical effect that is further disruptive with respect to lignin structures.

In another preferred embodiment of the method according to the invention, the water that is added 104 to the lignocellulosic biomass is heated beforehand to a temperature equal to or greater than 200°C (or another temperature suitable to obtain a mixture at a temperature comprised between 80 and 135°C), but kept in the liquid state. The resulting mixture has a temperature comprised between 80°C and 135°C, suitable for the subsequent step in which the mixture is maintained 106 at the temperature up to the subsequent vaporization step 107 (flash). During this incubation, a process of softening of the fibers and of increase of their porosity begins.

The incubation time during which the mixture is maintained 106 at the temperature is variable, preferably from a minimum of 0.2 hours to a maximum of 25 hours, which increases as the incubation temperature decreases.

When the superheated mixture in the liquid phase is subjected to reduced pressure, there is a sudden adiabatic vaporization of the water accompanied by a considerable increase in volume. This increase in volume, equal to several thousand times, is greater than the volume increase that is characteristic of current steam explosion processes. Accordingly, the water performs its disruptive action with respect to the lignin structure and as a consequence of this action the cellulose fibers are dispersed in the medium and partially deaggregated, and therefore can be attacked easily by the hydrolysis enzymes used in the subsequent processes.

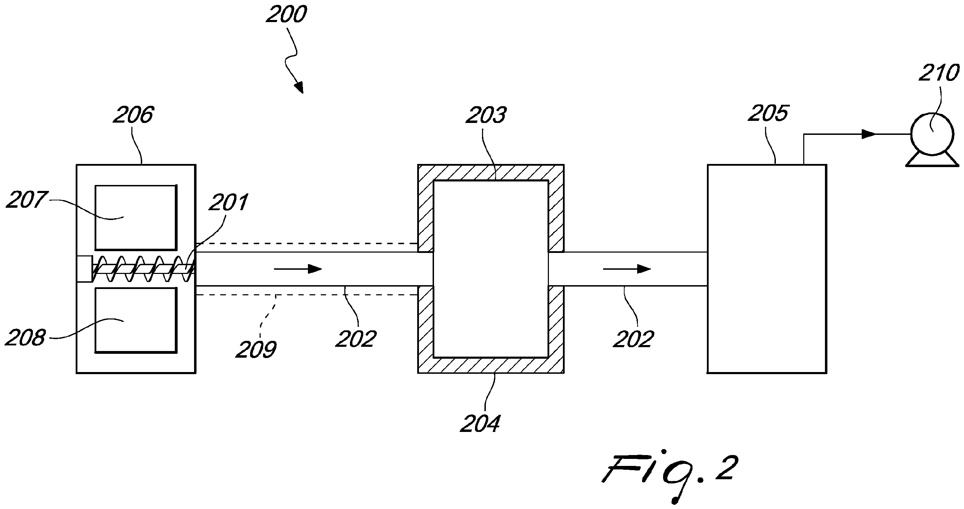

With reference to Figure 2, the present invention furthermore relates to an apparatus 200 for the treatment of a lignocellulosic biomass according to the method described previously, which comprises:
a) movement means 201 adapted to move the mixture comprising water and the lignocellulosic biomass in a conveyance duct 202 by applying a partial vacuum;
b) a cooking vessel 203 connected to the conveyance duct and comprising heating means 204 adapted to heat and maintain the temperature of the mixture comprising water and the lignocellulosic biomass;
c) a vessel at a pressure that is lower than atmospheric pressure 205, which is connected to the conveyance duct 202 downstream of the cooking vessel 203, adapted to vaporize the mixture comprising water and the lignocellulosic biomass.

It should be noted that the structure of the apparatus according to the invention is advantageously such as to allow a continuous treatment process which can be utilized directly for industrial applications.

In a preferred embodiment of the apparatus according to the invention, the movement means 201 comprise at least one volumetric pump, for example a volumetric screw pump.

In another preferred embodiment of the apparatus according to the invention, the heating means 204 associated with the cooking vessel 203 comprise interspaces which are external to the cooking vessel and through which hot fluids flow. The steady-state temperature is maintained by means of the application of interspaces through which heat recovery water flows pass and finally, where necessary, with specific heating fluids.

In another preferred embodiment of the apparatus according to the invention, the vessel at a pressure that is lower than atmospheric pressure 205 is kept at an absolute pressure comprised between 5 and 250 millibars (0.5 and 25 kPa). This pressure is achieved first of all by virtue of the condensation of the water vapor generated in vaporization and furthermore by virtue of the extraction of the residues of air which, together with other vapors, is entrained along the conveyance duct with the mixture of water and biomass being fed. In order to achieve the adequate vacuum in the vaporization (flash) vessel 205, all the steam formed by water vaporization is condensed quantitatively and the condensation is cooled to such a temperature as to obtain a vapor pressure that corresponds to the absolute pressure that is desired in 205; for example, to operate in 205 at the absolute pressure of 23 millibars, i.e., 2.3 kPa, the water vapor is condensed and the condensation is cooled to 20°C, because at this temperature the vapor pressure of the water is indeed 23 millibars (2.3 kPa). Furthermore, in order to ensure the reaching and stability of the vacuum the incondensable gas residues are extracted, for example with a suitable vacuum pump 210 (such as a liquid ring pump).

In a particularly preferred embodiment, the apparatus according to the invention further comprises a mixing vessel 206 which contains the lignocellulosic biomass, connected to the conveyance duct 202 upstream of the cooking vessel 203, which comprises input means 207 which are adapted to introduce water in the mixing vessel and mixing means 208 adapted to form said mixture comprising water and the lignocellulosic biomass.

In a particularly preferred embodiment of the apparatus according to the invention, the mixing vessel 206 is connected to said cooking vessel 203 by means of a portion of the connecting duct 202 provided with a heating jacket 209 that is crossed by hot fluids, for example the hot water that flows out of the process. This allows to perform or complete the heating of the biomass that is introduced in the cooking vessel 203.

In a particularly preferred embodiment, the apparatus according to the invention further comprises extraction means, associated with said mixing vessel 206 and adapted to remove the air from the lignocellulosic biomass, wherein said extraction means are preferably a vacuum pump.

In another particular preferred embodiment, the apparatus according to the invention further comprises milling means which are associated with the mixing vessel 206 and are adapted to mill the lignocellulosic biomass.

The lignocellulosic fibers pretreated in the apparatus described above with the method according to the invention can then be used in various hydrolysis and fermentation processes, for example for the production of alcohols, organic acids, biogases, feeds or amendments.

It should be noted that the cooking vessel 203 and the vaporization vessel 205 are kept at the respective steady-state pressures and the sudden vaporization of the water occurs in the transfer of the mixture from one vessel to the other.

With reference to the figures, the method and the apparatus according to the invention, designated respectively by the reference numerals 100 and 200, fully achieve the intended aim and objects.

In particular, the method according to the present invention is characterized by expansion ratios which are higher than those typically observed in steam explosion, without the need to generate steam but simply by heating the water kept liquid under pressure. Accordingly, the method provides important improvements with respect to the systems currently in use to separate cellulose fibers are from lignin casings:
- it saves energy, since it superheats the water instead of producing steam, i.e., it saves the latent heat of vaporization of the water;
- it uses quite lower heat levels, at the most 135°C, instead of 180-240°C of the background art, thus being able to utilize in the best possible way the enthalpies of the flows in output from the process;
- the heat levels adopted are low to the point that they can be met by common heat sources, such as for example remote heating or hot fluids that arrive from other processes. This entails advantages of an economic kind and furthermore reduces the environmental impact of the process;
- by virtue of the modest heat levels, the method allows moderate and well-managed cooking of the biomasses, with a limited degree of degradation of the molecules and therefore of byproducts that inhibit the fermentation stages.

The method and apparatus thus conceived are susceptible of numerous modifications and variations, all of which are within the scope of the appended claims; all the details may further be replaced with other technically equivalent elements.

In practice, the materials used, as well as the dimensions, may be any according to the requirements and the state of the art.

This application claims priority of Italian Patent Application No. 102017000098245.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A method (100) for the treatment of a lignocellulosic biomass comprising the steps of:
a) providing (101) a mixture comprising water and the lignocellulosic biomass having a water:biomass weight ratio lower than or equal to 2:1, preferably lower than 1:1;
b) heating (105) and maintaining (106) said mixture comprising water and the lignocellulosic biomass at a temperature comprised between 80°C and 135°C;
c) vaporizing (107) at an absolute pressure comprised between 5 and 250 millibar (0.5 and 25 kPa) said heated mixture comprising water and the lignocellulosic biomass.

2. The method according to claim 1, **characterized in that** said step of providing (101) the mixture comprising water and the lignocellulosic biomass comprises the steps of:
a1) milling (102) the lignocellulosic biomass;
a2) extracting (103) air from the milled lignocellulosic biomass;
a3) adding (104) water to the lignocellulosic mass from which the air has been extracted until a water:biomass weight ratio of less than 2:1, preferably less than 1:1, is obtained.

3. The method according to claim 2, **characterized in that** in step a1) the lignocellulosic biomass is milled into fragments smaller than 0.5 mm.

4. The method according to any one of the preceding claims, **characterized in that** in step b) said mixture is maintained at the temperature for a time comprised between 0.2 hours and 25 hours.

5. The method according to any one of the preceding claims, **characterized in that** the water of the mixture is desalinated water.

6. The method according to any one of the preceding claims, **characterized in that** the water of the mixture is carbonated.

7. The method according to any one of the preceding claims, **characterized in that** in said step of providing (101) the mixture comprising water and the lignocellulosic biomass the water is heated beforehand to a temperature equal to or higher than 200°C.

8. An apparatus (200) for the treatment of a lignocellulosic biomass according to the method of any one of the preceding claims, comprising:
a) movement means (201) adapted to move said mixture comprising water and the lignocellulosic biomass in a conveyance duct (202) by applying a partial vacuum;
b) a cooking vessel (203) connected to said conveyance duct and comprising heating means (204) adapted to heat and maintain the temperature of said mixture comprising water and the lignocellulosic biomass;
c) a vessel at a pressure that is lower than atmospheric pressure (205), which is connected to said conveyance duct (202) downstream of said cooking vessel (203), adapted to vaporize said mixture comprising water and the lignocellulosic biomass.

9. The apparatus according to claim 8, **characterized in that** the movement means (201) comprise at least one volumetric pump.

10. The apparatus according to any one of claims 8 and 9, **characterized in that** the heating means (204) comprise interspaces which are external to the cooking vessel and through which hot fluids flow.

11. The apparatus according to any one of claims 8-10, **characterized in that** said vessel at a pressure that is lower than atmospheric pressure (205) is kept at an absolute pressure comprised between 5 and 250 millibar (0.5 and 25 kPa).

12. The apparatus according to any one of claims 8-11, **characterized in that** it further comprises a mixing vessel (206) containing the lignocellulosic biomass, which is connected to said conveyance duct (202) upstream of said cooking vessel (203) and comprises input means (207) adapted to introduce water in the mixing vessel and mixing means (208) adapted to form said mixture comprising water and the lignocellulosic biomass.

13. The apparatus according to claim 12, **characterized in that** said mixing vessel (206) is connected to said cooking vessel (203) by means of a portion of the conveyance duct (202) provided with a heating jacket (209) through which hot fluids flow.

14. The apparatus according to claim 12 or 13, **characterized in that** it further comprises extraction means, which are associated with said mixing vessel and are adapted to remove air from the lignocellulosic biomass.

15. The apparatus according to any one of claims 12-14, **characterized in that** it further comprises milling means associated with said mixing vessel and adapted to mill the lignocellulosic biomass.

## Patentansprüche

1. Ein Verfahren (100) zur Behandlung einer Lignocellulosebiomasse, das folgende Schritte umfasst:
a) das Bereitstellen (101) einer Mischung, die Wasser und die Lignocellulosebiomasse umfasst und ein Gewichtsverhältnis von Wasser:Biomasse hat, das höchstens 2:1 und vorzugsweise weniger als 1:1 beträgt;
b) das Erhitzen (105) und Halten (106) der Mischung, die Wasser und die Lignocellulosebiomasse umfasst, bei einer Temperatur zwischen 80°C und 135°C;
c) das Verdampfen (107) bei einem absoluten Druck zwischen 5 und 250 millibar (0,5 und 25 kPa), wobei die erhitzte Mischung Wasser und die Lignocellulosebiomasse umfasst.

2. Das Verfahren gemäß Anspruch 1, **dadurch gekennzeichnet, dass** der Schritt des Bereitstellens (101) der Mischung, die Wasser und die Lignocellulosebiomasse umfasst, folgende Schritte umfasst:
a1) das Zerkleinern (102) der Lignocellulosebiomasse;
a2) das Extrahieren (103) von Luft aus der zerkleinerten Lignocellulosebiomasse;
a3) das Hinzufügen (104) von Wasser zu der Lignocellulosemasse, aus der die Luft extrahiert wurde, bis ein Gewichtsverhältnis von Wasser:Biomasse von weniger als 2:1, vorzugsweise weniger als 1:1, erreicht wurde.

3. Das Verfahren gemäß Anspruch 2, **dadurch gekennzeichnet, dass** in Schritt a1) die Lignocellulosebiomasse in Fragmente zerkleinert wird, die kleiner sind als 0,5 mm.

4. Das Verfahren gemäß einem beliebigen der obigen Ansprüche, **dadurch gekennzeichnet, dass** in Schritt b) die Mischung über einen Zeitraum zwischen 0,2 Stunden und 25 Stunden bei der Temperatur gehalten wird.

5. Das Verfahren gemäß einem beliebigen der obigen Ansprüche, **dadurch gekennzeichnet, dass** das Wasser der Mischung entsalztes Wasser ist.

6. Das Verfahren gemäß einem beliebigen der obigen Ansprüche, **dadurch gekennzeichnet, dass** das Wasser der Mischung carbonisiert ist.

7. Das Verfahren gemäß einem beliebigen der obigen Ansprüche, **dadurch gekennzeichnet, dass** in dem Schritt des Bereitstellens (101) der Mischung, die Wasser und die Lignocellulosebiomasse umfasst, das Wasser zuvor auf eine Temperatur gleich oder höher als 200°C erhitzt wird.

8. Eine Vorrichtung (200) für die Behandlung einer Lignocellulosebiomasse nach dem Verfahren gemäß einem beliebigen der obigen Ansprüche, das Folgendes umfasst:
a) Bewegungsmittel (201), ausgebildet, um die Mischung, die Wasser und die Lignocellulosebiomasse umfasst, durch Anlegen eines Unterdrucks in einer Förderleitung (202) zu transportieren;
b) ein Kochgefäß (203), das an die Förderleitung angeschlossen ist und Heizmittel (204) umfasst, ausgebildet, um die Mischung, die Wasser und die Lignocellulosebiomasse umfasst, zu erhitzen und ihre Temperatur aufrechtzuerhalten;
c) ein Gefäß mit einem Druck, der niedriger ist als der atmosphärische Druck (205), welches mit der Förderleitung (202) stromabwärts von dem Kochgefäß (203) verbunden und ausgebildet ist, um die Mischung, die Wasser und die Lignocellulosebiomasse umfasst, zu verdampfen.

9. Die Vorrichtung gemäß Anspruch 8, **dadurch gekennzeichnet, dass** die Bewegungsmittel (201) mindestens eine volumetrische Pumpe umfassen.

10. Die Vorrichtung gemäß einem beliebigen der Ansprüche 8 und 9, **dadurch gekennzeichnet, dass** die Heizmittel (204) Zwischenräume umfassen, die außerhalb des Kochgefäßes liegen und durch welche heiße Fluide strömen.

11. Die Vorrichtung gemäß einem beliebigen der Ansprüche 8-10, **dadurch gekennzeichnet, dass** das Gefäß bei einem Druck, der niedriger ist als der atmosphärische Druck (205), bei einem absoluten Druck zwischen 5 und 250 millibar (0,5 und 25kPa) gehalten wird.

12. Die Vorrichtung gemäß einem beliebigen der Ansprüche 8-11, **dadurch gekennzeichnet, dass** sie weiter einen Mischbehälter (206) umfasst, der die Lignocellulosebiomasse enthält und mit der Förderleitung (202) stromaufwärts von dem Kochgefäß (203) verbunden ist und Eingabemittel (207) umfasst, die ausgebildet sind, um Wasser in den Mischbehälter zu geben, und Mischmittel (208), die ausgebildet sind, um die Mischung zu bilden, die Wasser und die Lignocellulosebiomasse umfasst.

13. Die Vorrichtung gemäß Anspruch 12, **dadurch gekennzeichnet, dass** der Mischbehälter (206) mit dem Kochgefäß (203) über einen Abschnitt der Förderleitung (202) verbunden ist, der mit einem Heizmantel (209) ausgestattet ist, durch welchen heiße Fluide strömen.

14. Die Vorrichtung gemäß Anspruch 12 oder 13, **dadurch gekennzeichnet, dass** sie weiter Extraktionsmittel umfasst, die mit dem Mischbehälter verbunden und ausgebildet sind, um Luft aus der Lignocellulosebiomasse zu entfernen.

15. Die Vorrichtung gemäß einem beliebigen der Ansprüche 12-14, **dadurch gekennzeichnet, dass** sie weiter Zerkleinerungsmittel umfasst, die mit dem Mischbehälter verbunden und ausgebildet sind, um die Lignocellulosebiomasse zu zerkleinern.

## Revendications

1. Procédé (100) pour le traitement d'une biomasse lignocellulosique comprenant les étapes de :
a) fournir (101) un mélange comprenant de l'eau et la biomasse lignocellulosique ayant un rapport de poids eau:biomasse inférieur ou égal à 2:1, de préférence inférieur à 1:1 ;
b) chauffer (105) et maintenir (106) ledit mélange comprenant de l'eau et la biomasse lignocellulosique à une température comprise entre 80°C et 135°C ;
c) vaporiser (107) à une pression comprise entre 5 et 250 millibars (0,5 et 25 kPa) ledit mélange chauffé comprenant de l'eau et la biomasse lignocellulosique.

2. Procédé selon la revendication 1, **caractérisé en ce que** ladite étape de fournir (101) le mélange comprenant de l'eau et la biomasse lignocellulosique comprend les étapes de :
a1) broyer (102) la biomasse lignocellulosique ;
a2) extraire (103) de l'air de la biomasse lignocellulosique broyée ;
a3) ajouter (104) de l'eau à la masse lignocellulosique depuis laquelle l'air a été extrait jusqu'à ce qu'un rapport de poids eau:biomasse inférieur à 2:1, de préférence inférieur à 1:1, soit obtenu.

3. Procédé selon la revendication 2, **caractérisé en ce que** dans l'étape a1) la biomasse lignocellulosique est broyée en fragments plus petits que 0,5 mm.

4. Procédé selon l'une quelconque des revendications précédentes, **caractérisé en ce que** dans l'étape b) ledit mélange est maintenu à la température pendant un temps compris entre 0,2 heures et 25 heures.

5. Procédé selon l'une quelconque des revendications précédentes, **caractérisé en ce que** l'eau du mélange est de l'eau désalinisée.

6. Procédé selon l'une quelconque des revendications précédentes, **caractérisé en ce que** l'eau du mélange est carbonatée.

7. Procédé selon l'une quelconque des revendications précédentes, **caractérisé en ce que** dans ladite étape de fournir (101) le mélange comprenant de l'eau et la biomasse lignocellulosique l'eau est chauffée au préalable à une température supérieure ou égale à 200°C.

8. Dispositif (200) pour le traitement d'une biomasse lignocellulosique selon le procédé selon l'une quelconque des revendications précédentes, comprenant :
a) des moyens de déplacement (201) adaptés pour déplacer ledit mélange comprenant de l'eau et la biomasse lignocellulosique dans un conduit de convoyage (202) en appliquant un vide partiel ;
b) un récipient de cuisson (203) connecté audit conduit de convoyage et comprenant des moyens de chauffage (204) adaptés pour chauffer et maintenir la température dudit mélange comprenant de l'eau et la biomasse lignocellulosique ;
c) un récipient à une pression qui est inférieure à la pression atmosphérique (205), qui est connecté audit conduit de convoyage (202) en aval dudit récipient de cuisson (203), adapté pour vaporiser ledit mélange comprenant de l'eau et la biomasse lignocellulosique.

9. Dispositif selon la revendication 8, **caractérisé en ce que** les moyens de déplacement (201) comprennent au moins une pompe volumétrique.

10. Dispositif selon les revendications 8 et 9, **caractérisé en ce que** les moyens de chauffage (204) comprennent des interstices qui sont extérieurs au récipient de cuisson et à travers lesquels des fluides chauds s'écoulent.

11. Dispositif selon l'une quelconque des revendications 8-10, **caractérisé en ce que** ledit récipient à une pression qui est inférieure à la pression atmosphérique (205) est maintenu à une pression absolue comprise entre 5 et 250 millibars (0,5 et 25 kPa).

12. Dispositif selon l'une quelconque des revendications 8-11, **caractérisé en ce qu'**il comprend un récipient de mélange (206) contenant la biomasse lignocellulosique, qui est connecté audit conduit de convoyage (202) en amont dudit récipient de cuisson (203) et comprend des moyens d'entrée (207) adaptés pour introduire de l'eau dans le récipient de mélange et des moyens de mélange (208) adaptés pour former ledit mélange comprenant de l'eau et la biomasse lignocellulosique.

13. Dispositif selon la revendication 12, **caractérisé en ce que** ledit récipient de mélange (206) est connecté audit récipient de cuisson (203) au moyen d'une parie du conduit de convoyage (202) pourvue d'une chemise de chauffage (209) à travers laquelle des fluides chauds s'écoulent.

14. Dispositif selon la revendication 12 ou 13, **caractérisé en ce qu'**il comprend en outre des moyens de d'extraction, qui sont associés audit récipient de mélange et adaptés pour enlever de l'air de la biomasse lignocellulosique.

15. Dispositif selon l'une quelconque des revendications 12-14, **caractérisé en ce qu'**il comprend en outre des moyens de broyage associés audit récipient de mélange et adaptés pour broyer la biomasse lignocellulosique.
